# EUROPEAN PATENT SPECIFICATION

(11) **EP 1 476 548 B1**
(45) Date of publication and mention of the grant of the patent: **06.06.2007**
(21) Application number: 03704672.9
(22) Date of filing: 21.02.2003
(51) Int. Cl.: C12N 15/02, C12N 13/00, C12N 5/22

(54) **PROCESS OF CELL ELECTROFUSION**
VERFAHREN ZUR ELEKTROFUSION VON ZELLEN
PROCEDE D'ELECTROFUSION DE CELLULES

(30) Priority: 22.02.2002 EP 02290437
(43) Date of publication of application: 17.11.2004
(73) Proprietor: I.D.M. IMMUNO-DESIGNED MOLECULES, 75011 Paris (FR); CENTRE NATIONAL DE LA RECHERCHE SCIENTIFIQUE, 75794 Paris Cedex 16 (FR)
(72) Inventor: TESSIE, Justin, F-31520 Ramonville Saint Agne (FR); BARTHOLENS, Jacques, F-49730 Turquant (FR)
(74) Representative: Grosset-Fournier, Chantal Catherine
(86) International application number: PCT/EP2003/001798
(87) International publication number: WO 2003/070933

(56) References cited:
- EP-A- 1 130 088
- WO-A-89/10690

## Description

The present invention relates to a process of cell electrofusion.

More particularly, the present invention relates to the use of an electrical field applied to a cellular mixture for the preparation of a cell population enriched in cellular heterohybrids. It also relates to a method for the production of such cell population, and to a device designed to make use of this method. It also relates to a cell population obtained by using this method and the use of such cell population for the preparation of a pharmaceutical compound. It also relates to a device allowing the implementation of the method for the production of a cell population enriched in cellular heterohybrids.

The fusion of a somatic cell with another one can lead to the production of very interesting and useful hybrid cells (Lucy, 1977). Among the most developed type of heterohybrids are hybridomas obtained from the fusion of clonal B lymphocytes and myeloma cells, which are widely used for the production of monoclonal antibodies, since Kohler and Milstein publication in 1975.

Preclinical studies have shown that vaccines consisting of antigen presenting cells/tumor cells heterohybrids can provide effective active immunization against animal tumors and specific in vitro sensitization of T cells against relevant tumor antigens (Guo *et al.,* 1994, Gong *et al.,* 1997, Shu and Cohen, 2001).

Hybrids of autologous tumor and allogeneic dendritic cells that present antigens expressed by the tumor in concert with the co-stimulating capabilities of dendritic cells were generated. Some patients injected with such cellular preparation exhibited some regression of renal carcinoma tumors (Kugler et al, 2000). Hybrid cell vaccination was also shown to be a potentially effective cancer immune therapy of metastatic melanoma (Trefzer *et al.,* 2000).

Cellular homo- or heterohybrids may be obtained by the addition of some virus or chemical fusogenic agents, such as PolyEthyleneGlycol, said agents presenting the disadvantage to be potentially contaminating external compounds. Heterohybrids may also be obtained by electrofusion of the cells.

The electrofusion process comprises two steps: a creation of contact between partners and a destabilization of cell membranes, which is caused by electropulsation i.e. applying of an electrical field. The contact step may take place before or after membrane destabilization due to electric field, membrane destabilization necessitating control methods and following of the field conditions.

The cells in contact at the time of the pulse can fuse during the process of spontaneous membrane repair or resealing. The efficacy of such fusion has been analyzed after low intensity alternating electric fields and has been applied for obtaining of hybridomas between B cells and myeloma cells synthesizing antibodies. Lo and Tsong (1984) applied short intense direct current pulses so as to induce fusion, the recovery of hybridomas resulting from such fusion is very low as well as their viability.

Different protocols of electrofusion have been tested for the generation of hybrids, all characterized by an empirical manipulation (Scott-Taylor *et al.,* 2000; Hayashi et al., 2002). In order to establish a close cell-to-cell alignment, a first dielectrophoresis step may be assessed. Dielectrophoresis leads to an unspecific alignment of cells and possesses numerous limitations such as the necessity of use of a non ionic medium for the manipulation, no pH control, elevation of medium temperature, limitation of the volume and then of the number of treated cells and necessity for treatment chambers with complex geometry. Furthermore, this method does not favor the formation of cellular heterohybrids rather than homohybrids.

There is a need for a rapid, efficient and reproducible process allowing industrial application, in order to provide clinical grade cell preparations, approvable by regulatory authorities and containing a high proportion of cellular heterohybrids.

This is achieved by the present invention which relates to the use of an electrical field applied to a mixture containing a first type of cell (C1), a second type of cell (C2), a bispecific ligand able to bind to C1 and/or to C2 and non-covalent complexes formed between C1, C2 and the bispecific ligand, for the preparation of a cell population enriched in C1-C2 heterohybrids or for the preparation of C1-C2 heterohybrids.

The use of bispecific ligand increases the probability of formation of specific couple of cells C1 and C2, and thereby increases the yield of heterohybrids of interest.

One advantage of the invention is that the preformed couple of cells to be fused C1 and C2 do not need to be aligned along the electrical field direction before electrofusion. This results in less drastic conditions of electrical field and contact inducing treatment and wider choice of pulsing buffers than by dielectrophoretic means, thus decreasing the associated temperature increase, using buffers with appropriate physiological properties and accordingly less damage of cells.

By "type of cell" is meant cells having a defined function or characteristic, such as an antigen presenting cell, a lymphocyte or a tumor.cell originating from any tissue or any cellular preparation.

According to the present invention, the bispecific ligand which is added to antigen presenting cells and another type of the cells is used as target system only, and does not mediate substantially the internalization of the antigen with the APC.

The method according to the invention allows to prepare cellular heterohybrids, the increase of the yield of specific hybrid formation being obtained by incubating a first type of cells, a second type of cells and a bispecific ligand able to bind to C1 and to C2. C1, C2 and the ligand incubate in an adapted medium and at adapted relative concentration chosen so as to favor the binding of the ligand to the cells and the formation of non-covalent complexes. As heterologous cells are contacted via the bispecific ligand, the electrical field impulsion is followed by preferential formation of cellular heterohybrids, thus raising the yield of heterohybrids rather than homohybrids.

By "electrical field" is designated an electrical field applied by using any device and reaction conditions known by any man of the art specialized in the applying of electric field to cells so as to modify them while keeping them alive: The electrical field is applied as a succession of electrical impulses.

The term "mixture" designates a set of different types cells, ligands and non-covalent complexes resulting from the incubation of a first type of cells, a second type of cells and a bispecific ligand able to bind to the first and/or to the second type of cells, in conditions and for a time sufficient for the formation of non-covalent complexes. As in classical equilibrium conditions, different non covalent complexes are formed. There are binary complexes, comprising the ligand bound to C1 or to C2, and ternary complexes, being constituted by the association of the bispecific ligand to both C1 and C2. As formation of non-covalent complexes obeys to equilibrium conditions between free and associated partners, the mixture also contains the free ligand and unbound cells.

The appliance of an adapted electrical field to the mixture induces the fusion of cell membranes and results in the formation of cellular hybrids, and particularly C1-C2 heterohybrids.

The term "heterohybrid" designates a hybrid cell originating from the fusion of a first type of cells and a second type of cells. Heterohybrids involved in the invention are mainly bikaryons.

The term "cell population enriched in C1-C2 heterohybrids" designates the cell population resulting from the appliance of an electrical field to the mixture; it is enriched in C1-C2 heterohybrids, but also contains homohybrids and unfused cells.

When cellular heterohybrids are intended to be used for the treatment of a patient treatment, C1 and C2 may be either autologous (auto) or allogeneic (allo) cells relatively to the patient. Autologous cells may come from a sample taking, such as blood taking, or from a tissue biopsy. Allogeneic cells may come from a cell lineage, such as characterized lineages accessible from libraries such as American Tissue Culture Collection (USA). Therefore, different types of heterohybrids may be generated and
characterized relatively to the patient according to their auto/auto, auto/allo, allo/auto or allo/allo origin.

The present invention relates to a method for the production of a cell population enriched in C1-C2 heterohybrids comprising a step of applying an electrical field to a mixture containing a first type of cell (C1), a second type of cell (C2), a bispecific ligand able to bind to C1 and/or to C2 and non-covalent complexes formed between C1, C2 and the bispecific ligand, said electrical field being designed to induce cellular fusion, enabling the formation of heterohybrids.

As a general description, the method contains a first step of applying to a mixture such as above described an electrical field designed to induce cellular fusion and enabling formation of heterohybrids, and a second step of recovery of the cell population enriched in C 1-C2 heterohybrids.

In a particular embodiment of the invention, the method comprises a preliminary step of preincubation of C1, C2 and the bispecific ligand for a time sufficient for the formation of non-covalent complexes between C1, C2 and the ligand.

The cellular concentration of each partner of the mixture, i.e. of C1, C2, and of the bispecific ligand, is chosen relatively to the respective size of each partner population and to their respective equilibrium constants so as to favor the formation of complexes i.e. bikaryons. Incubation conditions, medium, and duration may be determined by a man skilled in the art as to favor the formation of complexes.

Cellular and ligand concentrations, incubation conditions and time necessary for the formation of non-covalent complexes may be determined by a man skilled in the art, according to relative size of cell populations and equilibrium constants defined for each partner of the complexes.

The term "affinity" designates a degree of interaction between a ligand and a receptor. The ligand may be for instance an antibody and the receptor may be for instance an antigen.

The term "equilibrium constant" designates the concentration of a ligand needed to occupy half of the receptors present. In case of bispecific ligand, two equilibrium constants shall have been defined, one for each class of receptor to which it may bind.

Among the numerous parameters, which are critical for the implementation of the present invention, there is the relative affinity of the bispecific ligand for each of the partner of the complex. Hence, the ratio of cellular concentration of C1 and C2 will be determined according to the equilibrium constant of the bispecific ligand for C1 and C2: the greater will be the equilibrium constant for a cell type, the lower will be the needed cellular concentration of the said cells.

The cell concentration of each type of cells in the reaction medium is preferably close to about 10⁶ to 10⁸ cells/ml, and preferably 10⁷ cells/ml so that the C1/C2 ratio is preferably close to 1. The optimal bispecific ligand concentration in the medium is to be calculated so as to favor the formation of bikaryons. It should be comprised between about 0.01 and 10 mg/100 ml of ligand for a total number of 10⁹ cells and preferably 0.1 to 1 mg/100 ml of ligand for 10⁹ cells.
According to this embodiment, the method comprises the following steps:
1- Putting in contact a first type of cell (C1), a second type of cell (C2) and a bispecific ligand able to bind to C1 and/or to the C2 for a time sufficient for the formation of non-covalent complexes between C1, C2 and the ligand. Alternatively putting in contact a first cell type (C1) and a bispecific ligand and then a second cell type (C2), the bispecific ligand being able to bind to C1 and/or C2 for a time sufficient for the formation of non-covalent complexes between C1, C2 and the ligand.
2- Applying to a mixture of step 1) an electrical field designed to induce cellular fusion and particularly heterohybrids, then after a time sufficient for membrane redistribution and cell fusion performed between about 20 to about 40°C (preferably between about 25 to about 37°C and more preferably at about 30°C).
3- Recovering the cell population enriched in C1-C2 heterohybrids

In a more particular embodiment of the invention, the mixture to which the electrical field is applied is in the form of a continuous flow.

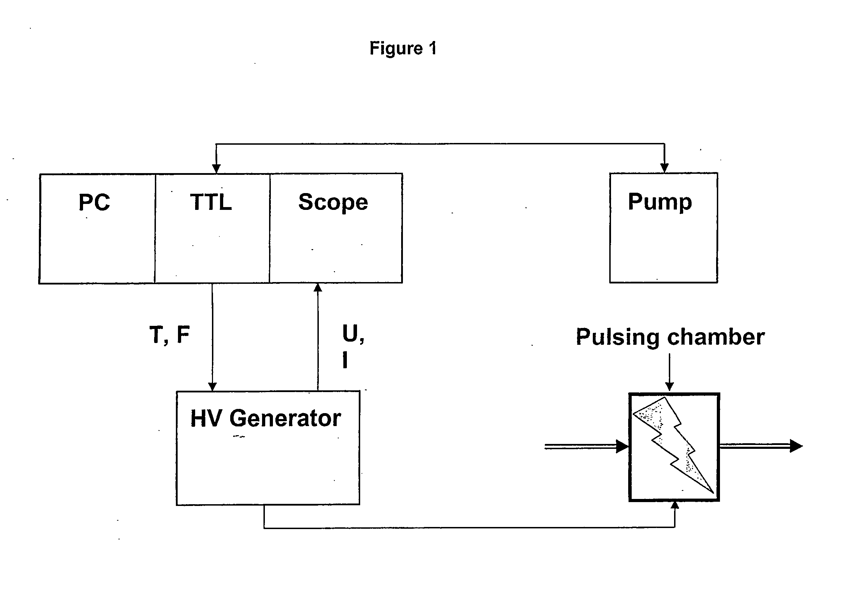

In an other particular embodiment of the invention, the mixture to which an electrical field is applied is in a form of a sequential flow (or batch), i.e., once a pulsing chamber (as defined on figure 1 for instance) is filled with the mixture, the flow is stopped and then the electrical field is applied before renewing the contents of the chamber.

Resident time of the mixture inside the electrical field must be set in such manner that whatever the form of the flow (continuous or sequential flow), it must last a time sufficient to allow accumulation of permeabilizing field conditions at surface of cells. The permeabilizing field is the field which induces a membrane potential difference locally greater than the value inducing a membrane destabilization. This parameter is a function of the size of the cell, of its shape, of its origin and of its physiological state. Permeabilizing field conditions result from the accumulation of positive and negative charges on opposite side of the surface of a cell due to the application of an electrical field.

The key parameters that must be determined to allow accumulation of permeabilizing field conditions at surface of cells are:
i) intensity of electrical field,
ii) number of pulses,
iii) duration of each pulse, and
iv) speed of the flow when electropulsation (or electrofusion) is achieved on a continuous flow.

Those parameters have to be ascertained for each type of cells to be fused, and a balance between parameters of each cell type is to be used to set the best conditions of electrofusion.

Setting the best value of the electrical field may be carried out by measuring percentage of permeabilized cells as a function of the electrical field value. The curves obtained allow to determine electrical field value for which about 100% of cells are permeabilized (E₁₀₀). Duration (t) and number (n) of pulses suitable for electrofusion are ascertained by measuring the percentage of permeabilized cells as a function of the duration of the pulses (nxt).

A mean for measuring percentage of permeabilized cells as a function of any preceding parameters is to use propidium iodide (PI). PI is a fluorescent dye that enters only in dead cells and that binds on nucleic acid inside nucleus of cells. After addition of this fluorescent dye a short time (about 10 minutes) before eletropulsation, the number of permeabilized cells is evaluated by detecting presence of fluorescent cells. The detection may be carried out with a Fluorescence Activating Cell Sorter (FACS) for instance, and the level of fluorescence is measured in arbitrary unit. Low fluorescence (ranging from about 2 to about 4 U.A. for instance) is indicative of non-permeabilized cells, high fluorescence (ranging from about 600 to about 10000 U.A. for instance) is indicative of dead cells and intermediate fluorescence (ranging from about 70 to about 100 U.A. for instance) is indicative of permeabilized cells having loaded the fluorescent dye. Hence, a fluorescent level may be considered low when it is about 30 to about 50 times lower than the intermediate fluorescence, which is considered as intermediate when it is about 6 to about 10 times lower than the higher fluorescence.

In case of sequential flow process, after having set the electric field intensity and the duration of pulses, cells are submitted to a given number of pulses of electric field. Electrodes must be parallel plates, parallel bars or parallel grids.

In case of continuous flow process, after having set the values of electrical field intensity, the pulse duration, the frequency of delivery is determined in order for the cells to be submitted to a given number of pulses at the chosen speed of the flow. In order to reach the best conditions of implementation of the present invention, an essential feature when carrying out electrofusion in continuous flow is to set the flow at such speed that the complex formed between C1, bispecific ligand and C2 has its greatest axis parallel to said electrical field. When a complex is formed between C1, C2 and a bispecific ligand, this complex may be viewed as 2 particles linked by a bar. The greatest axis of this complex is the line joining the center of the 2 cells and being parallel to the bar. If the electrical field is parallel to this greatest axis, then, when permeabilizing field conditions are reached, the negative charges present on one side of a cell are facing the positive charges present on the other cell. The attractiveness existing between the negative and the positive charges brings closest the two cells favoring the fusion of the cells. Due to the anisotropic form of the complex (i.e. having a length greater than its width), the complex tends to direct its greatest axis in the direction of the flow. The position of the complex in the flow is considered as the preferred direction of the complex. Accordingly, to obtain the best conditions of fusion, a device producing an electrical field parallel to the flow, and thus parallel to the greatest axis of the complex, has to be used and the speed of the flow must not exceed a threshold over which the flow looses its laminar regime and becomes turbulent thus disturbing the preferred direction of the complex. The flow Reynolds number must be below 2000. The Reynolds number is a non-dimensional parameter representing the ratio of the momentum forces to the viscous forces in fluid flow. The momentum represents a quantity of motion. Viscous forces are related to viscosity of the fluid, viscosity being the molecular property of a fluid which enables it to support tangential stresses for a finite time and thus to resist deformation (ratio of shear stress divided by shearing strain). The flow is a stream or movement of air or other fluid, or the rate of fluid movement, in the open or in a duct, pipe, or passage. All these parameters and definitions are well known from the man skilled in the art.

The speed of the flow may be for example of about 10 ml/min, and may be adapted according to the number of cells to be treated or to other necessities.

The flow rate is governed by the speed of the flow and the cross section of the pulsing chamber and may be adapted according to the number of cells to be treated or to other necessities (such as pulse frequency). It may be ranging from about 2 to about 75 ml/min in one pulsing chamber and is more preferably of about 10 ml/min. This allows the treatment of a larger volume of mixture than when applying the electrical field to a batch of medium containing cells. The speed of the flow and the resulting volume of cells exposed to electropulsation determine the number of fused cells. This is in contrast with the state of the art where the size of the electrofusion chamber in practice is inferior to 10 ml and thus severely limits the amount of produced heterohybrids. In the state of the art, when heterohybrids are intended to immunize a patient in need thereof, one or several millions of hybrids are required for each injection, so as to stimulate an efficient immune response. The preparation of many doses of efficient quantity of any specific heterohybrid, necessitating the treatment of several millions of cells by the method according to the invention is then possible.

In a more particular embodiment, the method of the invention comprises a step of applying to C1 and/or to C2 a treatment intended to kill or to block proliferation of the cells, before preincubation of C1, C2 and the bispecific ligand. In another embodiment of the invention, the method comprises a step of applying to C1-C2 heterohybrids a treatment intended to kill or to block proliferation of the cells after applying an electrical field designed to induce cellular fusion. Treatment may be applied using any protocol known by a man in the art and, such as, for example, γ-irradiation, at doses preferably comprised between about 150 and 200 Gray (Habal *et al.,* 2001, Kikuchi *et al.,* 2001). Cells may also be rendered non-tumorigenic by the appliance of an electrical field in which the duration, the frequency and the intensity of the electrical impulsion are designed to kill cells.

In a particular embodiment of the invention, the intensity of the electrical field applied to the cells is comprised from about 100 to about 4000 V/cm, and preferably from about 800 to about 2000 V/cm, and preferably from about 1200 to about 1800 V/cm and more preferably from about 1400 to about 1600 V/cm. These values correspond to electrical field designed for the destabilization of the cell membrane; a higher intensity is required for killing cells.

In an other particular embodiment of the invention, the duration of the electrical impulse is comprised from about 1 microsecond to about 100 millisecond, and preferably from about 20 to about 1000 microseconds, and preferably from about 50 to about 250 microseconds, and more preferably about 200.

In an other particular embodiment of the invention, the number of electrical impulses applied to a cell is comprised from about 1 to 100, and preferably from about 4 to 20, and more preferably about 5.

In an other particular embodiment of the invention, the electrical field is applied in a direction approximately parallel or approximately perpendicular to the flow, and preferably approximately parallel to the flow. The application of an electric field in a direction parallel to the flow appears to be less drastic for the cells. Furthermore, it necessitates a less intense electric field. Electrodes are built on the two sides of the pulsing chamber, the walls of which are made of an insulating biocompatible material. Their shapes can be either grids or rings.

When electrical field is applied perpendicular to the flow, electrodes can be either plates or bars.

In an other particular embodiment of the invention, cells are contained in a medium having an osmolarity comprised from about 150 to about 400 mOsm/kg, and more preferably from about 200 to about 400 mOsm/kg, and more preferably about 200 mOsm/kg. A lightly hypoosmolarity (about 200 mOsm/kg) brings a swelling of the cells and prevents surface undulations, which are known to act against a close cell membrane contact.

There is no need for the addition of any component, such as sucrose or other, that would be required to maintain cell integrity. This limits any potential external contamination and limits the number of components to be controlled and analyzed by regulatory authorities, and simplifies the process by using a fusion medium identical to the culture medium used for the preceding steps. As an example, AIM V medium (Gibco-Life Technologies) may be used. Therefore, it has a positive effect on fusion yield while preserving cell viability.

According to the method of the invention, the electrical impulse is unipolar or bipolar. In a more particular embodiment of the invention, the shape of the electrical impulse usable for the method is a square wave, a sinusoid, a triangle, or present exponential decline.

After being submitted to the process of electropulsation, cells present at their surface structural alterations resulting from destabilization, which spontaneously reseal. When two cells are brought close one to another, the step of resealing the structural alterations leads to the fusion of the two cells one with another. Hence, after going through the pulsing chamber, the mixture is left at rest, in order to allow the heterofusion to be achieved with high yield.

Means for measuring required time for resealing is to use propidium iodide (PI). This fluorescent dye is added at increasing time (ranging from 0 to 20 minutes for instance) after the last pulse, and the decreasing number of fluorescent cells in course of time is an index of the time required for resealing. The time needed for resealing all permeabilized cells may vary from one cell type to another cell type and is depending of the temperature at which the experiments are conducted (the more temperature decreases, the more time of resealing increases). The electrical treatment brings a local membrane fusion (membrane coalescence). The mixture must then be incubated at about 30 to about 37°C during about 30 minutes to about 4 hours and more preferably about 1 hours to obtain a cellular fusion, i.e. hybrid cell formation.

In a particular embodiment of the invention, the first type of cell (C1) submitted to the method is an antigen presenting cell, and preferably a dendritic cell or a macrophage.

Antigen presenting cells (APC) express high level of CMH Class I and class II as well as co-stimulatory molecules. The fusion of an APC with a second type of cell (C2) expressing some antigens on its surface allows the preparation of a cellular heterohybrid expressing both co-stimulatory molecules from APC and surface antigens from said second type of cell, said heterohybrid having therefore a higher immunogenicity than C2 alone.

Antigen presenting cells according to the invention may be dendritic cells (DCs), macrophages or B lymphocytes, as known by a man skilled in the art. DCs used may be immature or mature DCs, the higher level of expression of surface markers on mature DCs rendering them preferable.

In a particular embodiment of the invention, the antigen presenting cell is a monocyte-derived antigen presenting cell (MD-APC).

MD-APCs are obtainable by the culture of monocytes in presence of specific differentiation factors. As an example, the present invention may take advantage of MD-APCs produced according to methods described in WO 94/26875, WO 97/44441, US 5,662,899 or US 5,804,442 patent applications. Mature dendritic cells may be obtained according to methods known by a man skilled in the art, or according to methods described in Bocaccio *et al* (2002).

MD-APCs may be autologous or allogeneic to a patient to which the heterohybrids are intended to be administered. If allogeneic MD-APCs are to be used, they may be selected of the same haplotype as the one of the patient for at least the main determinants of class I subtype.

In an other particular embodiment of the invention, the second type of cell (C2) submitted to the method is a tumoral cell either alive or treated so as to be killed or detoxified.

Tumor cells may originate from a tumor lineage, possibly expressing known antigens, and available for example at ATCC or as lines derived from a patient tumor. Cells may also come from a fragment of tumor excised from the patient to be treated. Tumor cells may be killed before the fusion, by using to any method known by a man skilled in the art, such as γ-irradiation or applying an electrical field.

According to the invention, the bispecific ligand targets the first type of cells on the one hand and the second type of cells on the other hand.

When the first type of cells is constituted by antigen presenting cells, said possible antigen presenting cells targets for a ligand are, for example, IgG Fc receptors such as FcγRI (CD64), FcγRII (CD32), FcγRIII (CD16). In particular, due to its higher affinity for Fc, FcγRI (CD64) presence on the cell surface could be more favorable for inducing heterohybrids if, for example, IgG are used as bispecific ligands. FcaR (CD89) surface marker of APCs represents a possible ligand when IgA are used as bispecific ligand. The DC-sign receptor and the mannose receptors are also adequate targets when using oligosaccharadic lectins as binding ligands.

Other possible targets on the surface of antigen presenting cells are CD83, CD80, or CD86 molecules, when they are expressed, or adherence molecules such as ICAM-1 (CD54) or LFA 3 (CD-58).

When the second types of cells is constituted by tumors cells, possible targets on the surface of tumor cells are, for example:
- over-expressed membrane antigens, such as prostate PSA or PSMA, Her-2/neu, HSP-70, EGF-R, MUC-1, Melan-A, MART1, p53, CEA, NYESO, TRP-1, TRP-2, MAGEs, BAGEs, TAG72, GP-100, sialyl-Lewis oligosaccharides,
- selectine ligands, such as O-glycanes or gangliosides, which are glycanic antigens associated to cancer, in particular LH or MUC-1 antigens.
- viral antigens expressed by tumors, such as HBs, HCV, papillomavirus, HPV E6, E7.

In any case, a man skilled in the art is able to determine, by FACS or by any other mean, the presence on the tumor cell surface a known determinant and then is able to choose or to design an adapted bispecific ligand.

The bispecific ligand according to the invention may be any ligand having two parts, each of them having a specific affinity for one of the two types of cells. As an example, a bispecific ligand according to the invention may be a fusion protein, or a protein linked to a sugar, this last part being able to bind onto receptors located on the cellular surface. As a particular example, a bispecific ligand according to the invention may comprise one or several mannose group exhibiting affinity for mannose receptors located on antigen presenting cells.

In a particular embodiment of the invention, the bispecific ligand is an antibody or a bispecific antibody.

The bispecific ligand according to the invention may be an antibody, the Fc part of which is able to bind to Fc receptors located on the surface of one type of cell, the variable part being specific for an antigen located on the surface of the second type of cells. As IgG having an IgG1 isotype exhibit a higher affinity for Fc receptors, it is preferable to use IgG1 antibody. Cells expressing more particularly Fcγ receptors on their surface are dendritic cells and macrophages. In a specific embodiment of the invention, the bispecific ligand is an anti-tumor IgG1 antibody binding to the tumor cell via its variable region and to the antigen presenting cell high affinity Fc receptor through its Fc part.

The bispecific ligand may also be a bispecific antibody, comprising Fab1 and Fab2 fragments, each of these fragments binding to a specific ligand located on the surface of C1 for one part or C2 for the other part. More particularly, the bispecific ligand may bind to the antigen presenting cell via high affinity Fc receptor, without blocking the binding of IgG to Fc receptor (US patent 6,248,358).

In a more specific embodiment of the invention, the bispecific ligand binds to antigen-presenting cells via their surface Fc receptors, for one part, and to a tumor cell via cell surface antigens, for the other part.

The present invention relates to a method for the production of C1-C2 heterohybrids formed between a first type of cells (C1), a second type of cells (C2) and a bispecific ligand able to bind to C1 and/or to C2 comprising the step of preparation of a cell population enriched in C1-C2 heterohybrids and the subsequent isolation of C1-C2 heterohybrids.

Heterohybrids may be isolated from other components, and particularly from non-fused cells, and recovered by successive steps selecting on the presence of marker specific for C1 and another marker specific for C2. This can be done by any method known by a man skilled in the art, such as FACS sorting, differential centrifugation or magnetic beads selection. The non-fused cells may possibly be recycled to the pulsing chamber and submitted again to an electrical field, to increase the final yield of heterohybrids.

Also disclosed is a cell population enriched in C1-C2 heterohybrids such as obtained by applying a method as previously described.

The cell population is characterized in that it contains from about 60 to about 100 % of living cells. Cell viability may be characterized by cells permeability to a component such as Trypan blue or propidium iodide, or by assessing the functionality of cellular esterases. The cell preparation may be used as such. Another possibility is to freeze the cell preparation in order to store it before use. All methods and device to freeze, store and thaw cells are known from the man skilled the art.

The percentage of heterohybrids is comprised between about 15 and about 80 % of the recovered cells, and preferably from about 25 to about 50 % of the recovered cells.

Heterohybrids may be observed and quantified by any method known by a man skilled in the art, such as incubation with fluorescent antibodies specific for each of the two initial types of cells and microscopic observation, or by FACS sorting. The cell population may also contain cellular conjugates in which cells are still linked by the bispecific ligand.

The calculation of the percentage of heterohybrids is based on the number of heterohybrids in the cell population after fusion related to the number of cells initially present before fusion. In particular, under the assumption that only bikaryons are formed, a usable mathematical formula can be: (2N2) / (N1 + 2N2) x 100, with N1= number of cells with one nuclei, N2: number of cells with 2 nuclei.

A device for the reduction to practice of a method for the production of a cell population enriched in heterohybrids contains all elements required for production of heterohybrid cells in reproducible conditions. It is preferably a sterile closed system and of single use, it also can be automated under control of a dedicated software.

One aspect is a device allowing the application of an electrical field on a mixture under the form of continuous flow. The mixture contains the cells to be fused complexed to the bispecific ligand.

Another aspect is a device allowing the application of an electrical field on a mixture under the form of sequential flow. The mixture contains the cells to be fused complexed to the bispecific ligand.

In the present invention the use of device allowing the application of an electrical field on a continuous flow allows to increase drastically the amount of couple of cells liable to be submitted to the electrical field and to be fused.

Another advantage of the invention is that the combined use of cells C1 and C2 coupled by bispecific ligand and of a device allowing the application of an electrical field on a continuous flow act together to drastically increase the amount of heterohybrids of interest.

Such a device comprises at least one pulsing chamber (2) comprising at least two electrodes adapted to produce a substantially uniform field transverse to the flow passing between them, said electrodes being plate (15, 16) or bar (18, 19) disposed substantially parallel to each other in a plane substantially parallel to the flow traversing between the electrodes.

In a particular embodiment the device comprises at least two electrodes adapted to produce a substantially uniform field approximately parallel to the flow passing between them, said electrodes being in form of a grid (23, 24), a perforated disc or a ring (20, 21).

In a more particular embodiment, the device comprises at least two ring-shaped electrodes (20, 21) or perforated discs, in which the inner edges resulting from intersection of the vertical plan with the transversal plans may be rounded in order to produce a more homogeneous electrical field (thus producing an electrode more torus-shaped than ring-shaped).

When the electrodes are ring-shaped or disc-shaped, the ratio of the length of pulsing chamber to the inner diameter of said electrodes is greater than about 2.5, preferably ranging from about 2.5 to about 10, and more preferably equal to about 3.

The electrodes of the pulsing chamber (2) are made in stainless steel with a coating present to reduce electrochemical reactions in order to limit the release of electrochemical products from the electrodes. These products may be deleterious for the viability of cells. The coating may be made from gold, platinium or any other metal having similar or equivalent physico-chemical properties. Electrodes may nevertheless be made from stainless steel when bipolar pulses are applied with short delay between polarity inversion. Aluminium should be avoided.

The device comprises in addition to at least one pulsing chamber (2), a mixing chamber (1), an incubation chamber (3), feeding means (4, 5, 6, 10a) for feeding liquid partners of mixture into said mixing chamber (1), first connection means (7) for liquid communication between an outlet of said mixing chamber (1) and an inlet of said pulsing chamber (2), and second connection means (8) for liquid communication between an outlet of said pulsing chamber (2) and an inlet of said incubation chamber (3). Such arrangement befits for the implementation of the method of electropulsation on continuous flow.

According to one particular embodiment the feeding means comprise means for purging liquid, such as a syringe or a pipe (4, 5, 6 or 12) connected to a peristaltic pump (10a).

In a particular embodiment at least one of the connection means (7, 8) for liquid communication of the pulsing chamber (2) is equipped with closure means such as a tap (13, 14) or a clamp, allowing the closure of the pulsing chamber (2) once it is filled. The feeding means (4, 5, 6, 8) comprise means for purging liquid, such as a syringe or a pipe connected to a peristaltic pump (10a, 10b). The pipe (8) is connected to a second peristaltic pump (10b) to empty the pulsation chamber after the electrical treatment. Such arrangement befits for the method of electropulsation on sequential flow.

According to another particular embodiment the feedings means (4, 5, 6) may be equal to number of partners of mixture to mix. Alternatively those means may be connected to a multi-way tap (11) positioned before the mixing chamber. This multi-way tap (11) collects the different partners of the mixture, and is connected to an inlet of the mixing chamber (1). The mixing of the different partners of the mixture (C1, C2 and the bispecific ligand) reaches its equilibrium inside the mixing chamber and the mixture is transferred in a continuous flow or in a sequential flow toward the pulsing chamber (2).

In a particular embodiment the step of preincubation is achieved in a receptacle outer of the device used to achieve electropulsation. In an another embodiment, the preincubation is achieved in a mixing chamber (1) coupled with the pulsing chamber (2) of the device.

An outlet of the mixing chamber (1) is connected to an inlet of the pulsing chamber (2) by a feeding mean (7) which may be a pipe.

An outlet of the pulsing chamber (2) is connected to an inlet of the incubation chamber (3), by a feeding mean (8) which mean may be a pipe.

The incubation chamber (3) is connected by means of exit for a liquid (9) allowing to recover the mixture containing the cell population enriched in heterohybrids.

In another embodiment, the incubation chamber may be detached from the remainder of the device to recover the mixture containing the cell population enriched in heterohybrids in order to sort the heterohybrids of interest from the remainder of cell population. In another particular embodiment, the incubation chamber, once detached, may be connected to a device allowing the sorting out of heterohybrids of interest by means of cell surface markers such as a FACS.

All the means described above are designed to keep the mixture and heterohybrids of interest in sterile conditions.

In an advantageous embodiment the pulsing chamber (2) is connected to an incubation chamber (3) in which the cells recently submitted to the electrical field are left at rest in order to allow the resealing of alterations of membrane resulting from electropulsation process, the development of membrane fusion and the formation of heterohybrids. The mixture is recovered from the electropulsation towards the incubation chamber by means of a pipe (8).

In ,a particular embodiment, the device may comprise at least two pulsing chambers as defined above, those chambers being mounted in parallel between the mixing chamber and the incubation chamber. The only limit of the number of pulsing chamber which may be used in parallel depends of the power of the generator producing the electrical field.

The device may also comprise a way of cell segregation, usable after post-fusion incubation, in order to separate fused and non fused cells. It also may contain a connection allowing non-fused cells to flow through the pulsing chamber and be submitted again to the electric flow.

Also disclosed herein is a kit for the production of a cell population enriched in C1-C2 heterohybrids, said kit comprising bags, fusion chamber, post fusion chamber, connecting tubes, media and washing solutions and possibly a pump or any device allowing the flow of the C1, C2 and ligand mixture.

This kit may preferably be a single use kit forming a closed system and allowing the heterohybrid preparation according to industrial good manufacturing practices. This kits may contain bags, fusion chamber, post fusion chamber, connecting tubes, said elements being in plastic and/or plastic and metal.

The present application also discloses a pharmaceutical composition comprising at least, in association with a pharmaceutically acceptable vehicle, a cell population enriched in C1-C2 heterohybrids. It also concerns a pharmaceutical composition comprising at least, in association with a pharmaceutically acceptable vehicle, C1-C2 heterohybrids. Such pharmaceutical composition may allow the administration of a dose from about 10⁶ to about 10⁸ C1-C2 heterohybrids, and preferably about 10⁷ heterohybrids.

The present application also discloses the use of a cell population enriched in C1-C2 heterohybrids for the preparation of a drug either for immunization of a subject in need thereof or for the treatment of cancer. The hybrids may be used as therapeutic or as preventive vaccines. The hybrids may be totally autologous, totally allogeneic, or auto/allo relatively to the patient.

### LEGENDS OF THE FIGURES

**Figure 1: Scheme of the technology**
   The PC (Computer) controls the pulse duration, the frequency, the polarity inversion (if needed) and the pump flow rate. TTL (Transistor transistor Logic) signals are used. A manual control can be operated with simpler systems (pulse generators) when a PC is not available. The high voltage generator (HV generator), where the output voltage U is preset, supplies calibrated electric pulses of duration T on the electrodes of the pulsing chamber, which width is d. The field strength is E=U/d (with a flat parallel electrode technology). The voltage and current profiles of the pulses are monitored online with an oscilloscope (which may be a part of the PC). All data can be recorded on line when needed. "T" indicates "time", "F", "frequency" and "U", "Voltage intensity".

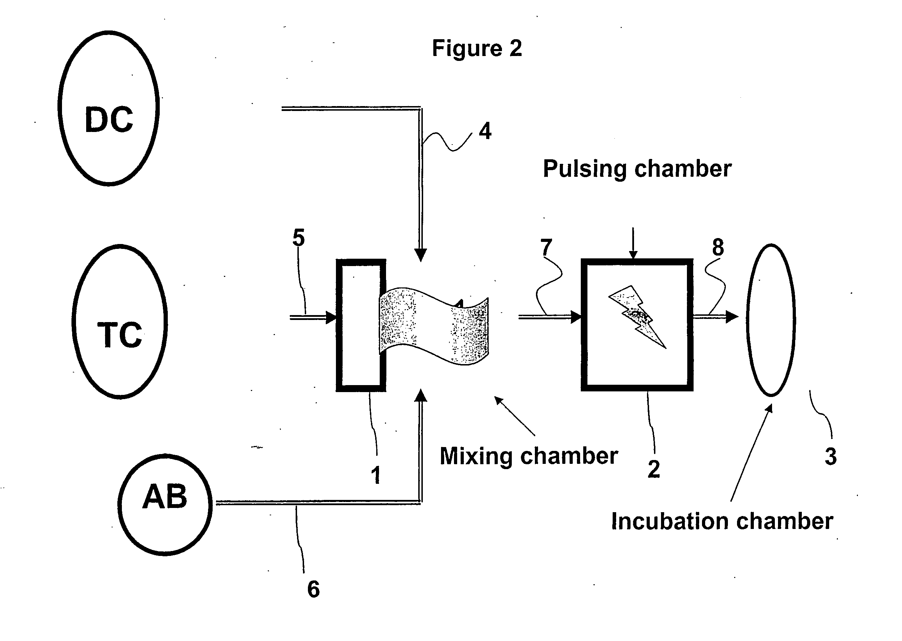

**Figure 2: Diagram of a fusion protocol on continuous flow.**
   DC, TC and AB represent the different fusion partners, contained in adapted pre-mix bags, being 1) the first type of cells (C1), 2) the second type of cells (C2) and 3) the bispecific ligand. In example 1, the first type of cells are dendritic cells (DC), the second type of cells are tumor cells (TC), and the bispecific ligand is an antibody (AB). The content of the three pre-mix bags is transferred to the mixing chamber (1) wherein the partners are mixed and pre-incubate, in order to create specific contacts between hetero-partners. After incubation, the mixture flows through the pulsing chamber (2), containing the two electrodes made in a biocompatible material and connected to the voltage generator (not shown). Well defined electric pulses (duration, intensity, number, polarity) are applied. The pulse frequency is controlled by a feed back of the flow and the chamber volume to give the well defined number of pulses. The mixture is transferred to a post-incubation chamber (3) in which cells are retained at 37°C for a time sufficient for stabilisation and reorganisation of the cell membrane (bikaryon formation), then the cell population is collected.

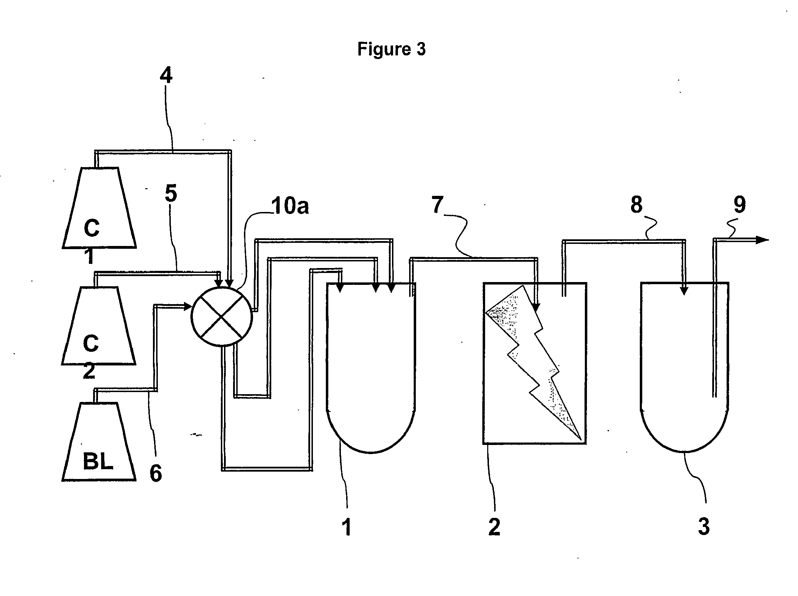

**Figure 3: Diagram of a fusion protocol allowing electrofusion on continuous flow.**
   C1, C2 and BL (Bispecific Ligand) represent the different fusion partners ,contained in adapted pre-mix bags, being 1) the first type of cells (C1), 2) the second type of cells (C2) and 3) the bispecific ligand. In example 1, the first type of cells are dendritic cells (DC), the second type of cells are tumoral cells (TC), and the bispecific ligand is an antibody (AB). The content of the three pre-mix bags is transferred to the mixing chamber (1), through feeding means (4, 5, 6) which are pipes in that case, connected to a peristaltic pump (10a) wherein the partners are mixed and pre-incubate, in order to create specific contacts between hetero-partners. After incubation, the mixture flows through the pulsing chamber (2) by mean of a pipe (7) connected to an outlet of the mixing chamber (1) and to an inlet of the pulsing chamber (2), containing the two electrodes made in a biocompatible material and connected to the voltage generator (not shown). Well defined electric pulses (duration, intensity, number, polarity) are applied. The pulse frequency is controlled by a feed back of the flow and the chamber volume to give the well defined number of pulses. The mixture is transferred to a incubation chamber (3) through a pipe (8) connected to an outlet of the pulsing chamber (2) and to an inlet of the incubation chamber (3) in which cells are retained at 37°C for a time sufficient for stabilization and reorganization of the cell membrane (bikaryon formation), then the cell population may be collected through a pipe (9).

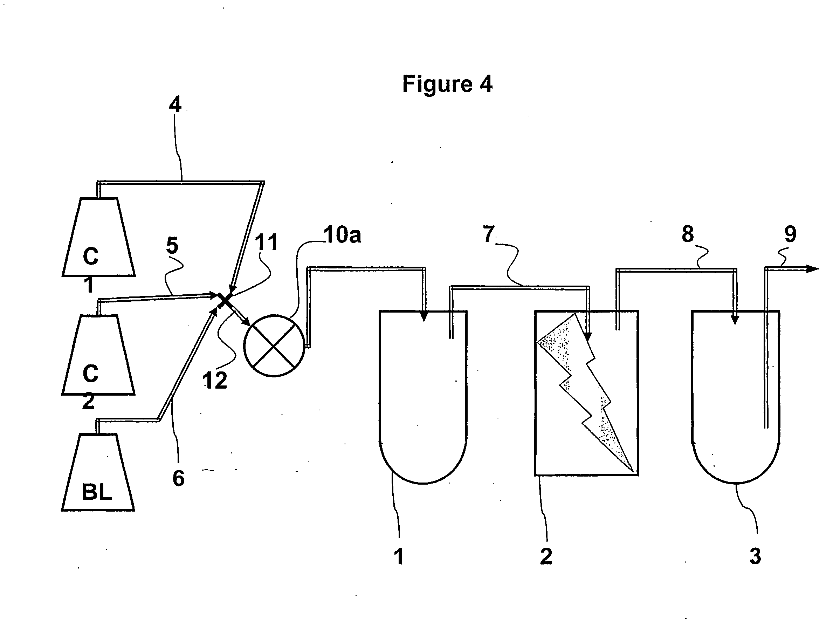

**Figure 4: Diagram of a fusion protocol allowing electrofusion on continuous flow.**
   Figure 4 is similar to figure 3, except that the pipes (4, 5, 6) are connected to a multiway-tap (11) collecting the content of the three pipes and transferring it into one pipe (12) connected to a peristaltic pump (10a) and then to the inlet of the mixing chamber (1).
**Figure 5: Diagram of a fusion protocol allowing electrofusion on sequential flow.**
   C1, C2 and BL (Bispecific Ligand) represent the different fusion partners ,contained in adapted pre-mix bags, being 1) the first type of cells (C1), 2) the second type of cells (C2) and 3) the bispecific ligand. In example 1, the first type of cells are dendritic cells (DC), the second type of cells are tumoral cells (TC), and the bispecific ligand is an antibody (AB). The content of the three pre-mix bags is transferred to the mixing chamber (1), through feeding means (4, 5, 6) which are pipes in that case, connected to a peristaltic pump (10a) wherein the partners are mixed and pre-incubate, in order to create specific contacts between hetero-partners. After incubation, the mixture is filled in the pulsing chamber (2) by mean of a pipe (7) which is equipped with a tap (13) connected to an outlet of the mixing chamber (1) and to an inlet of the pulsing chamber (2). The pulsing chamber (2) contains two electrodes made in biocompatible material and connected to the voltage generator (not shown). Well defined electric pulses (duration, intensity, number, polarity, frequency) are applied. The mixture is transferred to a incubation chamber (3) through a pipe (8) connected to an outlet of the pulsing chamber (2) and to an inlet of the incubation chamber (3) and which is equipped with a tap (14) and also connected to a peristaltic pump (10b). The cells are retained in incubation chamber (3) at 37°C for a time sufficient for stabilization and reorganization of the cell membrane (bikaryon formation), then the cell population may be collected through a pipe (9).

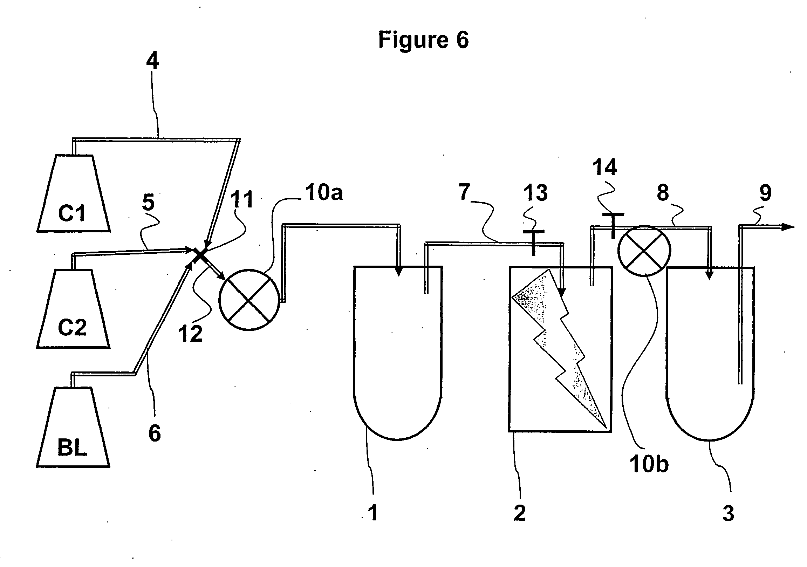

**Figure 6: Diagram of a fusion protocol allowing electrofusion on sequential flow.**

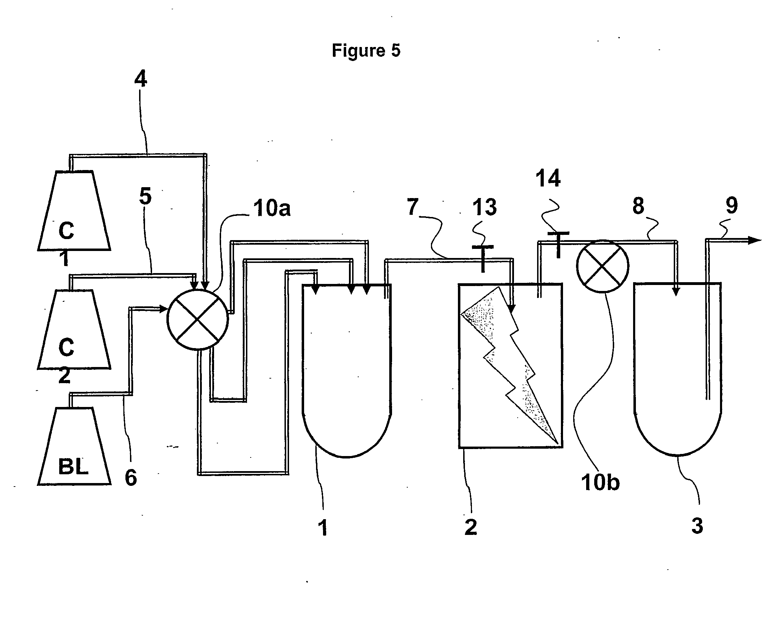

   Figure 6 is similar to figure 5, except that the pipes (4, 5, 6) are connected to a multiway-tap (11) collecting the content of the three pipes and transferring it into one pipe (12) connected to a peristaltic pump (10a) and then to the inlet of the mixing chamber (1).

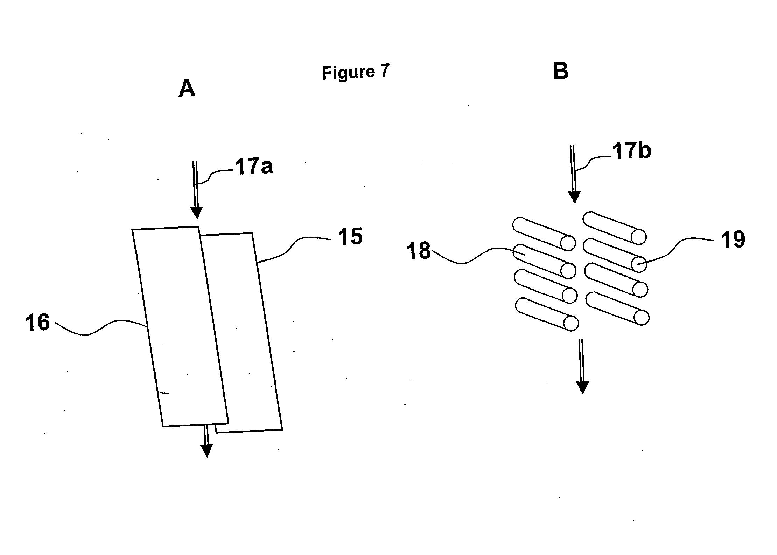

Figure 7: Scheme of a pulsing chamber producing an electrical field substantially transverse to the flow.
   Figure 7A and 7B exemplify shape and position of electrodes producing electrical field substantially transverse to the flow (indicated by arrows 17a and 17a). Figure 7A is indicative of plate-shaped electrodes (15, 16) and figure 7B is indicative of bar-shaped electrodes (18, 19). The electrodes are made in biocompatible material separated by a non-conductive material (not represented) and are positioned in a plan substantially parallel to the flow.

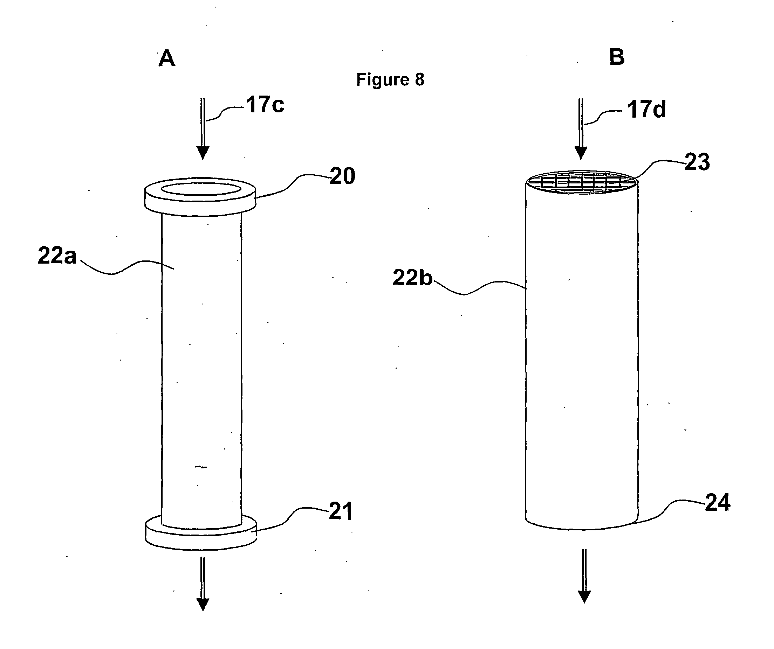

**Figure 8: Scheme of a pulsing chamber producing an electrical field substantially parallel to the flow.**
   Figure 8A and 8B exemplify shape and position of electrodes producing electrical field substantially parallel to the flow (indicated by arrows 17c and 17d). Figure 8A is indicative of ring-shaped (20, 21) electrodes and figure 8B is indicative of grid-shaped electrodes (23, 24). The electrodes are made in biocompatible material separated by a non-conductive material (22a and 22b) and are positioned in a plan substantially perpendicular to the flow.

### EXAMPLES

### Example 1: Production of hybrids from mature dendritic cells and tumoral cells.

### 1) Preparation of the fission partners

Immature monocyte derived dendritic cells (DCs) are obtained after 7 days of differentiation from peripheral blood mononuclear cells (PBMCs), according to a standardized process described in patent application WO 97/44441. Briefly, PBMCs are seeded at 5.10⁶ cells/ml in AIM V medium supplemented with 500 U/ml GM-CSF and 50 ng/ml IL-13 (complete AIM V). The culture is supplemented with 50 ng/ml of IL-13 on day 4.

Maturation of the dendritic cells is induced by a bacterial membrane extract, Ribomunyl® 1 µg/ml (Pierre Fabre Medicaments) and IFN-γ 500 U/ml (Imukin®, Boehringer Ingelheim) for 20 h. The final mature dendritic cells concentration is 10⁷ cells/ml in AIM V medium.

Tumor cells are human SKOV3 (ATCC Accession number HTB 77) cultivated in 25 cm² flasks in McCoy's medium (Gibco), supplemented in glutamine (Glutamax), penicillin, streptomycin (Gibco) and by 10% decomplemented Fetal Calf Serum (Gibco). During the culture, cell concentration is equal to 6.10⁴ cells/cm². 2.10⁶ SKOV3 cells are detached by trypsinisation EDTA from the culture and are washed twice in PBS. The final tumor cell concentration is 5.10⁶ cells/ml in AIM V medium.

Bispecific antibody MdX-210 (Medarex, Anandale, New Jersey) is able to recognize both FcγRI (CD64) on the surface of IFN-γ treated DCs and HER2/neu on the surface of SKOV3 cells. MdX-210 is used at a final concentration of 6 µg/ml within the mix chamber.

### 2) Electrofusion:

Two sterile pre-mix bags are connected to one mixing chamber, which in turn is connected to the electropulsation chamber. Bags and chambers are made in Ethylene Vinyl Acetate (EVA, Stedim, Aubagne, France). One of the pre-mix bags contains mature DCs (10⁷ cells/ml, in 200 ml of AIM V medium). The other pre-mix bag contains tumor cells (10⁷ cells/ml, in 200 ml of AIM V medium). The content of the two pre-mix bags is transferred to the mixing chamber, in which MdX 210 is added with a syringe so as to be present at a final concentration 6 µg/ml. DCs, tumor cells and bispecific antibody pre-incubate for 30 minutes at 20°C. The mix is then transferred to the electropulsation (or pulsing) chamber at a speed of 10 ml/min, which two electrodes are made in stainless steel with a coating present to reduce electrochemical reactions. The electric pulses (1,2 kV/cm, 0,1 ms, 10 times, square wave, 1 s interpulse delay) is applied, parallel to the flow of the cellular suspension through the electropulsation (or pulsing) chamber, at 20°C. The mixture is then transferred to the post-incubation chamber, in which cells are kept for one hour at 37°C in order to stabilize the hybrids. The cellular population is then recovered.

### 3) Characterization of the cells

Viability is assessed by Trypan blue exclusion, in order to evaluate cell integrity. Viable cells represent about 70 % of the total number of cells. The number of hybrid cells recovered is 6.10⁸.

Flow cytometry analysis is performed using a FACSCalibur with CellQuest Software (Becton Dickinson, San Jose, CA) by gating (FSC/SSC) the cell population of interest. Monoclonal antibodies anti-CD80, CD83, CD86, CD1a, HLA-ABC, HLA-DR are purchased from Immunotech (Marseille, France). PKH26, a red dye specific for tumor cell membranes, is used for characterization. Detection of Her-2/neu is assessed with FITC labeled murine anti- Her-2/neu antibodies.

Characterization shows that all heterohybrids exhibit on their surface typical DCs surface markers, CD80, CD83, CD86, CD1a, HLA-DR, with, for each marker, a relative fluorescence intensity of about half of the value of the fluorescence intensity on non-fused DCs. Heterohybrids also exibit Her-2/neu tumor marker on their surface.

### Example 2: Production of hybrids from macrophages and tumoral cells

### 1) Preparation of the fusion partners

Macrophages are obtained after 7 days of differentiation according to a standardized process described in WO 94/26875. Briefly, PBMCs are seeded at 5.10⁶ cells/ml in modified Dulbecco's medium supplemented with 500 U/ml GM-CSF and autologous serum. The final cellular concentration is 10⁷ cells/ml.

SKOV3 tumoral cells are prepared as described in example 1, at final concentration equal to 10⁷ cells/ml.

Bispecific ligand is MDX 210 (Medarex, Anandale, New Jersey), binding to FcγRI on the surface of MAKs and to HER2/neu surface antigen, located on SKOV3. MDX210 is used at a final concentration of 6 µg/ml within the mix chamber.

Electrofusion is achieved in reaction conditions identical to these described in example 1.

### 2) Results

Hybrids are characterized by a colocalization assay. Cells are labeled with PKH26, a red dye specific for tumor cell membranes, and with an anti-CD14 antibody bound to PC5 (green fluorochrome) (Becton-Dickinson). A 4 hours incubation is performed at 4°C. A confocal analysis is performed and shows the existence of heterohybrids. Cells viability is 60 %, the number of heterohybrids recovered is 8. 10⁸ cells. Heterohybrids exhibit the presence on their surface of the macrophage-specific marker CD64 as well as the Her-2/neu marker.

### Example 3: Production of hybrids from immature DCs and tumoral cells.

### 1) Preparation of the fusion partners

Immature monocyte derived dendritic cells (DCs) as described in example 1, after 7 days of differentiation from PBMCs, according to a standardized process described in patent application WO 97/44441. PBMCs are seeded at in AIM V medium supplemented with 500 U/ml GM-CSF and 50 ng/ml IL-13 (complete AIM V). The culture is supplemented with 50 ng/ml of IL-13 on day 4. The final concentration of immature dendritic cells is 10⁷ cells/ml in AIM V medium.

Tumor cells are human SKOV3 prepared as described in example 1. The final tumor cell concentration is 10⁷ cells/ml in AIM V medium.

Electrofusion is achieved in reaction conditions identical to these described in example 1.

### 2) Results

Characterization of the cell population recovered after fusion shows that the viability is 65% and 5.10⁸ heterohybrids are recovered. Heterohybrids exhibit both specific markers of immature dendritic cells, which are CD80, CD86, CD 1a, HLA-DR, and the presence of Her-2/neu tumor marker.

### Example 4: Production of hybrids from DCs and tumoral cells with a bispecific ligand being a sugar.

### 1) Preparation of the fusion partners

Immature DCs are obtained as in example 1. Tumor cells come from the LnCap prostate cell line (ATCC). The bispecific ligand is formed by a glycosynthon (oligomannose)-Lys-Lys-Lys-Lys-Ala-Cys, targeting the mannose receptor, linked via its terminal cystein to the Fab part of an antibody binding to TF (Thomsen and Friedenriech) pancarcinoma carbohydrate antigen present on tumors. Fusion is performed as in example 1.

### 2) Results

Viability and recovery of DC - LnCap heterohybrids are respectively 70% and 65%.

### Example 5: Production of hybrids from tolerogenic DCs and CD34⁺ cells

CD34⁺ to be injected to a patient in need thereof are collected from an allogeneic donor. In order to prevent or to diminish specifically the patient's immune reaction against grafted cells; it is desirable to pre-inject the patient with autologous tolerogenic dendritic cells fused to heterologous CD34⁺ cells.

### 1) Preparation of the fusion partners

Immature tolerogenic monocyte derived dendritic cells (DCs) are obtained after 7 days of differentiation from PBMCs, according to a process described in example 1. PBMCs are seeded at 5.10⁶ cells/ml in AIM V medium supplemented with 500 U/ml GM-CSF, 50 ng/ml IL-13 (complete AIM V) added with Dexamethasone 10⁻⁶ M. The culture medium is supplemented with 50 ng/ml of IL-13 on day 4.

CD34⁺ cells are isolated from apheresis product using the Isolex 300i Magnetic cell Selector (Nexell, Irvine, California). The purity of CD34⁺ cells selected to initiate the fusion is superior to 85 %.

The bispecific ligand is formed by an anti-CD34 antibody coupled to mannose, the antibody binding either to the CD34⁺ marker expressed on the surface of heterologous cells and to the mannose receptor located on autologous DCs. Fusion is performed as described in example 1. CD34⁺/autologous DCs hybrids are further selected using sorting by anti-CD34⁺ antibodies and by anti-CD89 antibodies.

### REFERENCES

Bocaccio et al. Identification of a clinical grade maturation factor for dendritic cells. *J. Immunother.,* 2002, 25(1),88-96.
Gong J., Dongshu Chen, Masashiro Kashiwaba, Donald Kufe. Induction of antitumor activity by immunization with fusions of dendritic and carcinoma cells. *Nature Medicine.* 1997; 3(5):558-561.
Guo Y, Wu M, Chen H, et al. 1994. Effective tumor vaccine generated by fusion of hepatoma cells with activated B cells. *Science* 263:518-520
Habal et al Ann Surgical Oncol. 2001 ; 8 :389-401
Hayashi T, Tanaka H, Tanaka J, Wang R, Averbook BJ, Cohen PA, Shu S., Immunogenicity and therapeutic efficacy of dendritic-tumor hybrid cells generated by electrofusion, *Clin Immunol,* 2002, 104 (1):14-20.
Kikuchi T, Akasaki Y, Irie M et al. 2001. Results of a phase I clinical trial of vaccination of glioma patients with fusions of dendritic and glioma cells. *Cancer Immuno. Immunother.* 50:337-344
Kugler A., G. Stuhler, P. Walden, G. Zöller, A. Zobywalski, P. Brossart, U. Trefzer, S. Ullrich, C. A. Müller, V. Becker, A. J. Gross, B. Hemmerlein, L. Kanz, G. A. Müller, R.-H. Ringert. Regression of human metastatic renal cell carcinoma after vaccination with tumor cell-dendritic cell hybrids. *Nature Medicine.* 2000; 6 (3):332-336.
Lucy J.A., Cell fusion. *TIBS,* 1977.
Scott-Taylor T.H., R. Pettengell, I. Clark, G. Stuhler, M.C. La Barthe, P. Walden, A.G. Dalgeish. Human tumor and dendritic cell hybrids generated by electrofusion : potential for cancer vaccines. *Biochimica & Biophysica Acta,* 2000, 265-279.
Shu S., Cohen. P. Tumor-dendritic cell fusion technology and Immunotherapy strategies. *J. of Immunotherapy,* 2001, 24 (2): 99-100.
Trefzer U., Weingart G., Yingwen Chien, Gunda Herberth, Adrian Karin, Helmut Winter, Audring Heike, Yajun Guo, Wolfram Sterry, Peter Walden. Hybrid cell vaccination for cancer immune therapy : first clinical trial with metastatic melanoma. *Int. J. Cancer.* 2000; 85, 618-626.
US 6,248,358 "Targeted immunostimulation with bispecific reagents", Romet-Lemomie et al.
WO 94/26875 "New macrophages, process for preparing the same and their use as active substances of pharmaceutical compositions.", Chokri et al.
US 5,662,899 "New macrophages, process for preparing the same and their use as active substances of pharmaceutical compositions.", Chokri et al.
WO 97/44441 "New antigen presenting cells, a process for preparing the same and their use as cellular vaccines." Chokri et al.
US 5,804,442 "Process for preparing macrophages, kits, and composition for the use of this process", Romet-Lemonne et al.

## Claims

1. Use of an electrical field applied to a mixture containing a first type of cell (C1), a second type of cell (C2), a bispecific ligand able to bind to C1 and/or to C2 and non-covalent complexes formed between C1, C2 and the bispecific ligand, for the preparation of a cell population enriched in C1-C2 heterohybrids or for the preparation of C1-C2 heterohybrids.

2. A method for production of a cell population enriched in C1-C2 heterohybrids comprising a step of applying an electrical field to a mixture containing a first type of cell (C1), a second type of cell (C2), a bispecific ligand able to bind to C1 and/or to C2 and non-covalent complexes formed between C1, C2 and the bispecific ligand, said electrical field, being designed to induce cellular fusion, enabling formation of heterohybrids.

3. A method according to claim 2 comprising a preliminary step of preincubation of C1, C2 and the bispecific ligand for a time sufficient for the formation of non-covalent complexes between C1, C2 and the ligand.

4. A method according to any one of claims 2 to 3, said method comprising the step of applying to C1 and/or to C2 a treatment intended to kill or to block proliferation of the cells, before preincubation of C1, C2 and the bispecific ligand

5. A method according to any one of claims 2 to 4, said method comprising the step of applying to C1-C2 heterohybrids a treatment intended to kill or to block proliferation of the hybrids after the applying of the electrical field designed to induce cellular fusion.

6. A method according to any one of claims 2 to 5, **characterized in that** said mixture to which an electrical field is applied is in a form of a sequential flow.

7. A method according to claim 6, **characterized in that** it comprises steps of adjusting intensity of said electrical field, adjusting number, and duration of pulse(s) of the said electrical field.

8. A method according to any one of claims 2 to 5, **characterized in that** said mixture to which an electrical field is applied is in a form of a continuous flow.

9. A method according to claim 8 **characterized in that** it comprises steps of adjusting speed of said continuous flow, intensity of said electrical field, adjusting number, duration and frequency of pulse(s) of the said electrical field in order to deliver a given number of electrical field pulses on the mixture.

10. A method according to claims 8 or 9 **characterized in that** the electrical field is applied to the mixture in a direction approximately parallel or approximately perpendicular to the flow, and preferably approximately parallel to the flow.

11. A method according to claim 9 or 10 **characterized in that** the step of adjusting said speed of the said continuous flow allows further to a complex formed between C1, bispecific ligand and C2 to have its greatest axis parallel to said electrical field.

12. A method according to anyone of claims 9 to I 1 **characterized in that** said speed of said continuous flow inside said electrical field is adjusted in order to stay preferably in laminar regime.

13. A method according to any one of claims 2 to 12 **characterized in that** intensity of said electrical field is comprised from about 100 to about 4000 V/cm, and preferably from about 800 to about 2000 V/cm, and more preferably from about 1200 to 1800 V/cm and more preferably from about 1400 to about 1600 V/cm.

14. A method according to any one of claims 2 to 13 **characterized in that** duration of said electrical field is comprised from about 1 microsecond to about 100 millisecond, and preferably from about 20 to about 1000 microseconds, and more preferably from about 50 to about 250 microseconds.

15. A method according to any one of claims 2 to 14 **characterized in that** number of electrical impulses applied to said mixture is comprised from about 1 to 100, and preferably from about 4 to 20.

16. A method according to any one of claims 2 to 15 **characterized in that** C1, C2 and the mixture are contained in a medium having an osmolarity comprised from about 150 to about 400 mOSmlkg, more preferably from about 200 to about 400 mOsm/kg, and more preferably about 200 mOsm/kg.

17. A method according to any one of claims 2 to 16 **characterized in that** the electrical impulse is unipolar or bipolar.

18. A method according to any one of claims 2 to 17 **characterized in that** the shape of the electrical impulse is a square wave, a sinusoid, a triangle, or with exponential decline.

19. A method according to any one of claims 2 to 18 **characterized in that** said mixture is left, after being submitted to said electrical field, at rest a time sufficient to allow formation of hybrids, said sufficient time ranging from about 30 minutes to about 4 hours, preferably about 1 hours.

20. A method according to any one of claims 2 to 19 **characterized in that** the first type of cell is an antigen presenting cell, and preferably a dendritic cell or a macrophage.

21. A method according to any one of claim 2 to 20 **characterized in that** the first type of cell is a monocyte-derived antigen presenting cell.

22. A method according to any one of claim 2 to 21 **characterized in that** the second type of cell is a tumor cell, either alive or treated so as to be killed or detoxified.

23. A method according to any one of claims 2 to 22 **characterized in that** the bispecific ligand is an antibody or a bispecific antibody.

24. A method according to claim 22 or 23 **characterized in that** the bispecific ligand binds to said antigen presenting cell via high affinity Fc receptors, for one part, and to said tumor cell via a cell surface antigen, for the other part.

25. A method for the production of C1-C2 heterohybrids formed between a first type of cells (C1), a second type of cells (C2) and a bispecific ligand able to bind to C1 and/or to C2 comprising a step of preparation of a cell population enriched in C1-C2 heterohybrids, according to any one of claims 2 to 24, and subsequent isolation of C1-C2 heterohybrids.

## Patentansprüche

1. Verwendung eines elektrischen Feldes, das an eine Mischung angelegt wird, die einen ersten Zelltyp (C1), einen zweiten Zelltyp (C2), einen zur Bindung an C1 und/oder C2 befähigten bispezifischen Liganden und zwischen C1, C2 und dem bispezifischen Liganden gebildete nicht-kovalente Komplexe enthält, zur Herstellung einer an C1-C2-Heterohybriden angereicherten Zellpopulation oder zur Herstellung von C 1-C2-Heterohybriden.

2. Verfahren zur Herstellung einer an C1-C2-Heterohybriden angereicherten Zellpopulation, umfassend einen Schritt des Anlegens eines elektrischen Feldes an eine Mischung, die einen ersten Zelltyp (C1), einen zweiten Zelltyp (C2), einen zur Bindung an C1 und/oder C2 befähigten bispezifischen Liganden und zwischen C1, C2 und dem bispezifischen Liganden gebildete nicht-kovalente Komplexe enthält, wobei das elektrische Feld so ausgelegt ist, dass es Zellfusionierung induziert, wobei die Bildung von Heterohybriden ermöglicht wird.

3. Verfahren nach Anspruch 2, das einen vorausgehenden Schritt der Vorinkubation von C1, C2 und dem bispezifischen Liganden für eine Zeit, die für die Bildung nicht-kovalenter Komplexe zwischen C1, C2 und dem Liganden ausreichend ist, beinhaltet.

4. Verfahren nach einem der Ansprüche 2 bis 3, wobei das Verfahren den Schritt der Anwendung einer Behandlung auf C1 und/oder C2 zum Abtöten oder Blockieren der Proliferation der Zellen vor der Vorinkubation von C1, C2 und dem bispezifischen Liganden beinhaltet.

5. Verfahren nach einem der Ansprüche 2 bis 4, wobei das Verfahren den Schritt der Anwendung einer Behandlung auf C1-C2-Heterohybride zum Abtöten oder Blockieren der Proliferation der Hybride nach dem Anlegen des elektrischen Feldes, das zum Induzieren der Zellfusionierung ausgelegt ist, beinhaltet.

6. Verfahren nach einem der Ansprüche 2 bis 5, **dadurch gekennzeichnet, dass** die Mischung, an die ein elektrisches Feld angelegt wird, die Form eines sequentiellen Flusses hat.

7. Verfahren nach Anspruch 6, **dadurch gekennzeichnet, dass** es Schritte der Einstellung der Intensität des elektrischen Feldes, Einstellung der Zahl und Dauer der (des) Pulse(s) des elektrischen Feldes beinhaltet.

8. Verfahren nach einem der Ansprüche 2 bis 5, **dadurch gekennzeichnet, dass** die Mischung, an die ein elektrisches Feld angelegt wird, die Form eines kontinuierlichen Flusses hat.

9. Verfahren nach Anspruch 8, **dadurch gekennzeichnet, dass** es Schritte der Einstellung der Geschwindigkeit des kontinuierlichen Flusses, der Intensität des elektrischen Feldes, Einstellung der Zahl, Dauer und Frequenz der (des) Pulse(s) des elektrischen Feldes beinhaltet, um eine vorgegebene Anzahl elektrischer Feldpulse an die Mischung abzugeben.

10. Verfahren nach einem der Ansprüche 8 oder 9, **dadurch gekennzeichnet, dass** das elektrische Feld an die Mischung in einer Richtung angelegt wird, die ungefähr parallel oder ungefähr senkrecht zum Fluss ist, vorzugsweise ungefähr parallel zum Fluss.

11. Verfahren nach Anspruch 9 oder 10, **dadurch gekennzeichnet, dass** der Schritt der Einstellung der Geschwindigkeit des kontinuierlichen Flusses ferner ermöglicht, dass ein zwischen C1, bispezifischem Liganden und C2 gebildeter Komplex seine größte Achse parallel zu dem elektrischen Feld hat.

12. Verfahren nach einem der Ansprüche 9 bis 11, **dadurch gekennzeichnet, dass** die Geschwindigkeit des kontinuierlichen Flusses innerhalb des elektrischen Feldes eingestellt wird, um vorzugsweise in einem laminaren System zu bleiben.

13. Verfahren nach einem der Ansprüche 2 bis 12, **dadurch gekennzeichnet, dass** die Intensität des elektrischen Feldes etwa 100 bis etwa 4000 V/cm, vorzugsweise etwa 800 bis etwa 2000 V/cm, bevorzugter etwa 1200 bis 1800 V/cm und bevorzugter etwa 1400 bis etwa 1600 V/cm ist.

14. Verfahren nach einem der Ansprüche 2 bis 13, **dadurch gekennzeichnet, dass** die Dauer des elektrischen Feldes etwa 1 Mikrosekunde bis etwa 100 Millisekunden, vorzugsweise etwa 20 bis etwa 1000 Mikrosekunden, bevorzugter etwa 50 bis etwa 250 Mikrosekunden ist.

15. Verfahren nach einem der Ansprüche 2 bis 14, **dadurch gekennzeichnet, dass** die Zahl der elektrischen Impulse, die auf die Mischung angewendet werden, etwa 1 bis 100, vorzugsweise etwa 4 bis 20 ist.

16. Verfahren nach einem der Ansprüche 2 bis 15, **dadurch gekennzeichnet, dass** C1, C2 und die Mischung in einem Medium enthalten sind, das eine Osmolarität von etwa 150 bis etwa 400 mOsm/kg, bevorzugter etwa 200 bis etwa 400 mOsm/kg und bevorzugter etwa 200 mOsm/kg hat.

17. Verfahren nach einem der Ansprüche 2 bis 16, **dadurch gekennzeichnet, dass** der elektrische Impuls unipolar oder bipolar ist.

18. Verfahren nach einem der Ansprüche 2 bis 17, **dadurch gekennzeichnet, dass** die Form des elektrischen Impulses Rechteck, Sinus, Dreieck oder mit exponentiellem Abfall ist.

19. Verfahren nach einem der Ansprüche 2 bis 18, **dadurch gekennzeichnet, dass** man die Mischung, nachdem sie dem elektrischen Feld ausgesetzt wurde, für eine Zeit ruhen lässt, die ausreichend ist, um die Bildung von Hybriden zu ermöglichen, wobei diese ausreichende Zeit im Bereich von etwa 30 Minuten bis etwa 4 Stunden, vorzugsweise etwa 1 Stunde, liegt.

20. Verfahren nach einem der Ansprüche 2 bis 19, **dadurch gekennzeichnet, dass** der erste Zelltyp eine Antigen-präsentierende Zelle ist, vorzugsweise eine dendritische Zelle oder ein Makrophage.

21. Verfahren nach einem der Ansprüche 2 bis 20, **dadurch gekennzeichnet, dass** der erste Zelltyp eine Monozyten-abgeleitete Antigen-präsentierende Zelle ist.

22. Verfahren nach einem der Ansprüche 2 bis 21 **dadurch gekennzeichnet, dass** der zweite Zelltyp eine Tumorzelle ist, entweder lebend oder behandelt, sodass sie abgetötet oder entgiftet ist.

23. Verfahren nach einem der Ansprüche 2 bis 22 **dadurch gekennzeichnet, dass** der bispezifische Ligand ein Antikörper oder ein bispezifischer Antikörper ist.

24. Verfahren nach Anspruch 22 oder 23, **dadurch gekennzeichnet, dass** der bispezifische Ligand einerseits über hochaffine Fc-Rezeptoren an die Antigen-präsentierende Zelle und andererseits über ein Zelloberflächenantigen an die Tumorzelle bindet.

25. Verfahren zur Herstellung von C1-C2-Heterohybriden, die zwischen einem ersten Zelltyp (C1), einem zweiten Zelltyp (C2) und einem zur Bindung an C1 und/oder C2 befähigten bispezifischen Liganden gebildet werden, umfassend einen Schritt der Herstellung einer an C1-C2-Heterohybriden angereicherten Zellpopulation gemäß einem der Ansprüche 2 bis 24 und anschließende Isolierung von C1-C2-Heterohybriden,

## Revendications

1. Utilisation d'un champ électrique appliqué à un mélange contenant un premier type de cellule (C1), a second type de cellule (C2), un ligand bispécifique capable de créer une liaison avec C1 et/ou C2 et des complexes non-covalents formés entre C1, C2 et le ligand bispécifique, pour la préparation d'une population cellulaire enrichie en hétérohybrides C1-C2 ou pour la préparation d'hétérohybrides C1-C2.

2. Procédé de production d'une population cellulaire enrichie en hétérohybrides C1-C2 comprenant une étape d'application d'un champ électrique à un mélange contenant un premier type de cellule (C1), un second type de cellule (C2), un ligand bispécifique capable de créer une liaison avec C1 et/ou C2 et des complexes non-covalents formés entre C1, C2 et le ligand bispécifique, ledit champ électrique étant adapté pour induire une fusion cellulaire permettant la formation d'hétérohybrides.

3. Procédé selon la revendication 2, comprenant une étape préliminaire de préincubation de C1, C2 et du ligand bispécifique pendant un temps suffisant pour la formation de complexes non-covalents entre C1, C2 et le ligand.

4. Procédé selon l'une quelconque des revendications 2 ou 3, ledit procédé comprenant l'étape d'application à C1 et/ou à C2 d'un traitement destiné à détruire ou bloquer la prolifération des cellules, avant la préincubation de C1, C2 et du ligand bispécifique.

5. Procédé selon l'une quelconque des revendications 2 à 4, ledit procédé comprenant l'étape d'application aux hétérohybrides C1-C2 d'un traitement destiné à détruire ou bloquer la prolifération des hybrides après l'application du champ électrique adapté pour induire la fusion cellulaire.

6. Procédé selon l'une quelconque des revendications 2 à 5, **caractérisé en ce que** ledit mélange auquel un champ électrique est appliqué, est sous la forme d'un flux séquentiel.

7. Procédé selon la revendication 6, **caractérisé en ce qu'**il comprend des étapes de réglage de l'intensité dudit champ électrique, de réglage du nombre et de la durée de(s) impulsion(s) dudit champ électrique.

8. Procédé selon l'une quelconque des revendications 2 à 5, **caractérisé en ce que** ledit mélange auquel un champ électrique est appliqué, est sous la forme d'un flux continu.

9. Procédé selon la revendication 8, **caractérisé en ce qu'**il comprend des étapes de réglage de la vitesse dudit flux continu, de l'intensité dudit champ électrique, de réglage du nombre, de la durée et de la fréquence de(s) impulsion(s) dudit champ électrique afin de délivrer un nombre donné d'impulsions de champ électrique sur le mélange.

10. Procédé selon les revendications 8 ou 9, **caractérisé en ce que** le champ électrique est appliqué au mélange dans une direction sensiblement parallèle ou sensiblement perpendiculaire au flux et de préférence sensiblement parallèle au flux.

11. Procédé selon la revendication 9 ou 10, **caractérisé en ce que** l'étape de réglage de la vitesse dudit flux continu permet en outre à un complexe formé entre C1, le ligand bispécifique et C2 d'avoir son axe le plus grand parallèle audit champ électrique.

12. Procédé selon l'une quelconque des revendications 9 à 11, **caractérisé en ce que** ladite vitesse dudit flux continu à l'intérieur dudit champ électrique est réglée afin de maintenir de préférence un régime laminaire.

13. Procédé selon l'une quelconque des revendications 2 à 12, **caractérisé en ce que** l'intensité dudit champ électrique est comprise d'environ 100 à environ 4000 V/cm et de préférence d'environ 800 à environ 2000 V/cm et de façon plus préférée d'environ 1200 à 1800 V/cm et de façon encore plus préférée d'environ 1400 à environ 1600 V/cm.

14. Procédé selon l'une quelconque des revendications 2 à 13, **caractérisé en ce que** la durée dudit champ électrique est comprise d'environ 1 microseconde à environ 100 millisecondes et de préférence d'environ 20 à environ 1000 microsecondes et de façon préférentielle d'environ 50 à environ 250 microsecondes.

15. Procédé selon l'une quelconque des revendications 2 à 14, **caractérisé en ce que** le nombre des impulsions électriques appliquées audit mélange est compris d'environ 1 à 100, et de préférence d'environ 4 à 20.

16. Procédé selon l'une quelconque des revendications 2 à 15, **caractérisé en ce que** C1, C2 et le mélange sont contenus dans un milieu ayant une osmolarité comprise d'environ 150 à environ 400 mOSm/kg, et de préférence d'environ 200 à environ 400 mOSm/kg et de façon préférentielle environ 200 mOSm/kg.

17. Procédé selon l'une quelconque des revendications 2 à 16, **caractérisé en ce que** l'impulsion électrique est unipolaire ou bipolaire.

18. Procédé selon l'une des revendications 2 à 17, **caractérisé en ce que** la forme de l'impulsion électrique est une onde carrée, une sinusoïde, un triangle, ou avec un déclin exponentiel.

19. Procédé selon l'une quelconque des revendications 2 à 18, **caractérisé en ce que** ledit mélange est laissé, après avoir été soumis audit champ électrique, au repos un temps suffisant pour permettre la formation d'hybrides, ledit temps suffisant étant compris d'environ 30 minutes à environ 4 heures et de préférence environ 1 heure.

20. Procédé selon l'une quelconque des revendications 2 à 19, **caractérisé en ce que** le premier type de cellule est une cellule présentatrice d'antigène et de préférence une cellule dendritique ou un macrophage.

21. Procédé selon l'une quelconque des revendications 2 à 20, **caractérisé en ce que** le premier type de cellule est une cellule présentatrice d'antigène dérivé d'un monocyte.

22. Procédé selon l'une quelconque des revendications 2 à 21, **caractérisé en ce que** le second type de cellule est une cellule tumorale, soit vivante, soit traitée afin d'être morte ou détoxifiée.

23. Procédé selon l'une quelconque des revendications 2 à 22, **caractérisé en ce que** le ligand bispécifique est un anticorps ou un anticorps bispécifique.

24. Procédé selon l'une des revendications 22 ou 23, **caractérisé en ce que** le ligand bispécifique crée une liaison avec ladite cellule présentatrice de l'antigène au moyen de récepteurs Fc de haute affinité, d'une part, et avec ladite cellule tumorale au moyen d'un antigène de surface cellulaire, d'autre part.

25. Procédé pour la préparation d'hétérohybrides C1-C2 formés entre un premier type de cellules (C1), un second type de cellules (C2) et un ligand bispécifique capable de créer une liaison avec C1 et/ou C2 comprenant une étape de préparation d'une population cellulaire enrichie en hétérohybrides C1-C2, selon l'une quelconque des revendications 2 à 24 et l'isolement subséquent d'hétérohybrides C1-C2.
